# FASCICULE DE BREVET EUROPEEN

(11) **EP 2 881 141 B1**
(45) Date de publication et mention de la délivrance du brevet: **01.06.2016**
(21) Numéro de dépôt: 14186897.6
(22) Date de dépôt: 29.09.2014
(51) Int. Cl.: A61N 1/375, A61N 1/05, A61N 1/372

(54) **Capsule intracardiaque implantable sur une paroi mince, notamment la paroi septale**
Intrakardiale Kapsel, die in eine sehr dünne Wand, insbesondere die Septumwand, implantierbar ist
Implantable intracardiac capsule on a thin wall, in particular the septal wall

(30) Priorité: 04.12.2013 FR 1362125
(43) Date de publication de la demande: 10.06.2015
(73) Titulaire: Sorin CRM SAS, 92140 Clamart Cedex (FR)
(72) Inventeur: Ollivier, Jean-François, 91190 Villiers le Bacle (FR)
(74) Mandataire: Dupuis-Latour, Dominique

(56) Documents cités:
- US-A1- 2004 215 301
- US-A1- 2009 082 828
- US-A1- 2013 268 042
- US-B1- 6 409 674

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif. L'invention concerne plus particulièrement ceux de ces dispositifs qui se présentent sous forme d'une capsule autonome implantable dans une cavité cardiaque (ventricule, oreillette ou même cavité cardiaque gauche artérielle), ci-après désignée "capsule autonome" ou "capsule *leadless".* Ces capsules sont dépourvues de toute liaison physique à un dispositif principal implanté (tel qu'un boitier de générateur d'impulsions de stimulation) ou non implanté (périphérique externe tel que programmateur ou dispositif de *monitoring* pour le suivi à distance du patient). Elles sont qualifiées pour cette raison de *leadless,* pour les distinguer des électrodes ou des capteurs disposés à l'extrémité distale d'une sonde (*lead*) conventionnelle, parcourue sur toute sa longueur par un ou plusieurs conducteurs reliant par voie galvanique l'électrode ou le capteur à un générateur qui est connecté à une extrémité opposée, proximale, de la sonde.

On notera toutefois, comme on le comprendra à la lecture de la description, que le caractère autonome de la capsule n'est pas intrinsèquement une caractéristique nécessaire de l'invention, et que cette dernière peut être aussi bien appliquée à des capsules montées de façon permanente à l'extrémité distale d'une sonde.

Pour leur maintien en place après implantation, les capsules *leadless* sont pourvues d'un organe d'ancrage, le plus souvent constitué d'une vis hélicoïdale saillante prolongeant axialement le corps de la capsule et destinée à pénétrer dans le tissu cardiaque par vissage au site d'implantation, de la même manière que pour les sondes à vis conventionnelles.

Le EP 2 394 695 A1 (Sorin CRM SAS) décrit un tel type de capsule *leadless* à vis, ainsi qu'un accessoire permettant son implantation au site choisi, par accostage de la vis axiale et entrainement en rotation de la capsule pour fixer celle-ci de façon permanente à la paroi cardiaque, où elle se trouvera maintenue par la vis axiale d'ancrage.

Il a été proposé des systèmes comparables mettant en oeuvre des aiguilles, des crochets, des barbes, etc., ces organes d'ancrage prolongeant la capsule à son extrémité distale de manière à pouvoir pénétrer dans les tissus et y assujettir de façon permanente la capsule à partir de cette extrémité.

En ce qui concerne la capsule proprement dite, celle-ci est généralement cylindrique d'une longueur de 20 à 40 mm dans le cas des capsules *leadless,* cette forme cylindrique étant imposée par la voie d'accès via le réseau veineux périphérique.

La configuration que l'on vient de décrire (forme cylindrique et organe d'ancrage axial) est compatible avec une implantation dans la zone de l'apex, en fond de ventricule, mais peut engendrer un certain nombre de difficultés pour d'autres sites d'implantation, par exemple sur le septum interventriculaire ou interauriculaire.

En effet, la faible largeur de la cavité ventriculaire droite, combinée aux battements cardiaques, peut provoquer un contact répété délétère de la partie proximale de la capsule avec la paroi libre du muscle cardiaque, la valve tricuspide ou encore les piliers.

Par ailleurs, la zone de l'apex n'est pas toujours la meilleure cible d'implantation pour certaines thérapies, et peut présenter un risque du fait de la faible épaisseur de la paroi dans les zones voisines, créant un risque non négligeable de perforation par l'organe d'ancrage au moment de la manoeuvre d'implantation.

Une autre difficulté encore tient au fait que l'accessoire d'implantation (comme celui décrit dans le EP 2 394 695 A1 précité) place généralement la capsule avec son axe principal dans le prolongement d'un cathéter ou d'un mandrin de l'accessoire, de manière à pouvoir passer par le réseau veineux périphérique et diriger la capsule jusqu'au site d'implantation. Lorsqu'il s'agit d'orienter la capsule vers l'apex du ventricule, un tel accessoire est tout à fait adapté, et permet d'atteindre de façon précise et parfaitement sécurisée le site d'implantation choisi, puis d'y ancrer la capsule et enfin désolidariser cette dernière d'avec l'accessoire d'implantation.

En revanche, si l'on souhaite implanter la capsule par exemple sur la paroi septale ou la paroi de l'oreillette droite, pour y ancrer la vis il est nécessaire d'aborder perpendiculairement cette paroi avec la capsule. Ceci implique de disposer d'un système à tête orientable, d'autant plus délicat à manipuler qu'avec les battements du coeur la tête de la capsule n'est pas naturellement dirigée vers le site d'implantation, bien au contraire, du fait de la forme tubulaire allongée de la capsule. Pour réduire ce risque, des capsules de forme aplatie ont été proposées, par exemple par le EP 2 537 555 A1 (Sorin CRM SAS), dont l'implantation est toutefois plus complexe que celle des capsules tubulaires allongées, car leur forme n'est pas naturellement adaptée au passage dans un vaisseau.

L'un des buts de l'invention est de remédier à ces divers inconvénients, en proposant une capsule de forme tubulaire allongée, notamment de type *leadless,* spécifiquement adaptée à une implantation sur le septum interventriculaire ou toute autre paroi, y compris les parois minces ou épaisses des différentes cavités cardiaques.

Essentiellement, la capsule de l'invention comporte un corps tubulaire combiné à une vis hélicoïdale d'ancrage. Mais au lieu de disposer la vis axialement dans le prolongement de la capsule à l'extrémité distale de celle-ci, cette vis est enroulée autour du corps tubulaire, le long de ce dernier. Dans sa configuration finale, la capsule se trouvera donc accolée à cette paroi, c'est-à-dire que l'axe du corps tubulaire de la capsule sera sensiblement parallèle à la paroi, au lieu de se présenter approximativement perpendiculairement à celle-ci comme dans le cas des capsules conventionnelles à vis axiale saillante. La capsule selon l'invention sera maintenue contre la paroi par coincement des tissus entre les spires de la vis et le corps de la capsule, le long d'une génératrice de cette dernière. On connaît certes, notamment d'après les US 2013/0268042 A1 et US 6 409 674 B1, des capsules munies d'une vis d'ancrage de grand diamètre qui est partiellement enroulée autour du corps de la capsule. Mais ces capsules sont destinées à être ancrées perpendiculairement (et non parallèlement) à la paroi, selon la configuration classique d'implantation. La capsule est munie à cet effet à son extrémité distale d'une pointe, d'un harpon et/ou de barbes permettant de faire pénétrer cette extrémité du corps dans la paroi et de l'y maintenir en place. La partie de vis enroulée autour du corps n'a alors qu'un rôle subsidiaire, pour renforcer le maintien de la partie du corps qui est enfoncée dans la paroi.

Plus précisément, l'invention propose une capsule intracardiaque comprenant, d'une manière en elle-même connue par exemple d'après le US 2013/0268042 A1 précité : un corps cylindrique ; une vis d'ancrage hélicoïdale solidaire du corps cylindrique, apte à pénétrer dans un tissu d'une paroi d'une cavité du coeur ; et, à une extrémité proximale du corps cylindrique, des moyens de solidarisation temporaire, en rotation et en translation, de la capsule à un accessoire d'implantation. La vis d'ancrage hélicoïdale entoure le corps cylindrique sur au moins une partie de la longueur de celui-ci, cette partie formant une région de contact destinée à venir en contact avec la paroi de la cavité du coeur. Sur l'étendue de la région de contact, le diamètre externe du corps cylindrique est inférieur au diamètre interne de la vis d'ancrage hélicoïdale, de manière à laisser subsister un intervalle libre entre le corps cylindrique et la vis d'ancrage hélicoïdale. La vis d'ancrage hélicoïdale est solidarisée au corps cylindrique au voisinage de l'extrémité proximale de la région de contact, et elle s'étend librement depuis cette extrémité proximale jusqu'à l'extrémité distale opposée.

De façon caractéristique de l'invention, ladite région de contact s'étend le long d'une génératrice du corps cylindrique et l'extrémité distale du corps cylindrique comporte une surface arrondie atraumatique.

Selon diverses caractéristiques subsidiaires avantageuses :
- la vis d'ancrage ne s'étend pas au-delà de l'extrémité de la surface arrondie atraumatique en direction axiale ou bien, au contraire, s'étend au-delà de cette extrémité sur l'étendue d'une ou deux spires de vis ;
- la surface arrondie atraumatique est conformée de manière à laisser exposée l'extrémité de la vis d'ancrage sur une longueur d'une ou deux spires frontales, de manière à pouvoir amorcer le vissage dans la paroi de la cavité du coeur par ces spires exposées ;
- l'intervalle libre subsistant entre le corps cylindrique et la vis d'ancrage hélicoïdale le long de la région de contact est compris entre 0,1 et 1 mm ;
- le corps cylindrique comprend en outre au voisinage de l'extrémité proximale de la région de contact un épaulement de butée dont le diamètre est supérieur à celui de la région de contact, l'intervalle libre s'étendant en direction distale à partir de cet épaulement de butée ;
- la capsule comprend en outre au moins une électrode sectorielle de détection/stimulation disposée sur le corps cylindrique, de préférence au voisinage de l'épaulement de butée ;
- la vis d'ancrage, hélicoïdale est une vis en matériau électriquement conducteur recouvert d'un revêtement électriquement isolant à l'exception d'au moins une zone localement dénudée formant électrode de détection/stimulation ;
- l'intervalle libre est un intervalle constant sur la longueur de la région de contact, ou bien un intervalle variable sur la longueur de la région de contact, cet intervalle étant accru côté proximal ;
- le pas de la vis d'ancrage hélicoïdale est un pas constant, ou bien un pas variable, ce pas étant réduit côté proximal.

On va maintenant décrire un exemple de mise en oeuvre de l'invention, en référence aux dessins annexés où les mêmes références désignent d'une figure à l'autre des éléments identiques ou fonctionnellement semblables.
La Figure 1 est une vue en élévation d'une capsule selon l'invention.
La Figure 2 est un détail de la partie centrale de la capsule, montrant la configuration d'une électrode de stimulation.
La Figure 3 est une vue perspective de la capsule de l'invention, prête à l'implantation.
La Figure 4 est une vue montrant la phase initiale d'accostage de la capsule contre la paroi cardiaque au moment de la manoeuvre d'implantation, avec l'extrémité de l'accessoire d'implantation.
La Figure 5 est homologue de la Figure 4, montrant la capsule dans sa position finale, implantée, après retrait de l'accessoire d'implantation.

On va maintenant décrire un exemple de réalisation de l'invention.

Sur les figures, la référence 10 désigne de façon générale une capsule *leadless,* qui se présente sous la forme d'un élément tubulaire allongé comprenant un corps cylindrique 12 avec une partie distale 14 et une partie proximale 16.

La partie proximale 16 porte des moyens de solidarisation temporaire, en rotation et en translation, de la capsule à un accessoire d'implantation (non représenté sur cette figure). Ces moyens 18 peuvent par exemple comprendre une tige lisse axiale sur laquelle viendra s'emmancher un mécanisme débrayable de liaison à un mandrin ou un sous-cathéter permettant d'entrainer en rotation la capsule. Un tel mécanisme débrayable simple est par exemple décrit dans la demande française FR 13 56020 du 24 juin 2013, au nom de Sorin CRM SAS, pour *"Capsule intracardiaque et accessoire d'implantation* in situ *par voie fémorale".* Essentiellement, ce mécanisme est constitué par un ressort hélicoïdal utilisé en compression radiale, c'est-à-dire pour son effet de striction, et non pour son effet de traction/compression axiale. Un tel ressort joue à la fois un rôle de moyen de liaison débrayable et de limiteur de couple à l'encontre d'une action de vissage excessive qui pourrait entraîner un "carottage" des tissus.

La capsule 10 comprend également une vis d'ancrage hélicoïdale 20 qui, de façon caractéristique de l'invention, est enroulée autour du corps cylindrique 12 sur une partie de la longueur de la capsule, partie dénommée "région de contact" 22. Côté proximal, la vis est solidarisée au corps tubulaire 12, par exemple par serrage des spires 24 les plus proximales sur une région 26 du corps tubulaire, prolongeant côté proximal la région de contact 22. La région 24 présente un diamètre accru par rapport à celui de la région de contact 22, de manière à former un épaulement de butée 28 définissant la limite proximale de la région de contact 22.

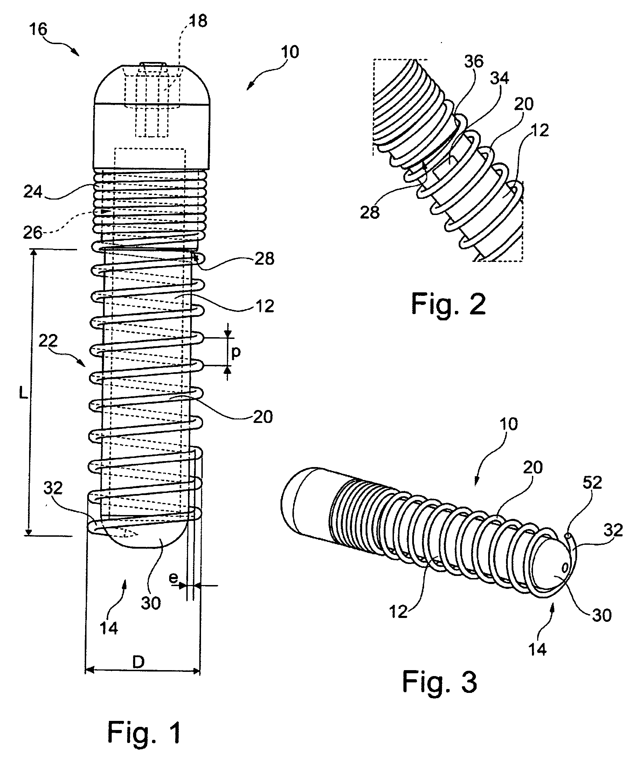

La vis d'ancrage hélicoïdale 20 est libre sur toute l'étendue de la région de contact 22, jusqu'à son extrémité distale 14 du corps cylindrique. Cette extrémité distale 14 comporte une surface arrondie atraumatique 30. Dans l'exemple illustré la vis 20 ne s'étend pas au-delà de l'extrémité de cette surface 30 en direction axiale, mais d'autres géométries de vis peuvent être envisagées, notamment avec une ou deux spires dépassant de la surface arrondie 30. La forme arrondie de la surface 30 permet toutefois de laisser exposée l'extrémité 32 de la vis 20, sur une longueur de l'ordre d'une ou deux spires frontales (comme cela est mieux visible sur la Figure 3), ce qui permettra d'amorcer le vissage dans les tissus par ces spires exposées, comme on le décrira plus loin.

Le diamètre hors tout D de la capsule, correspondant au diamètre extérieur de la vis d'ancrage hélicoïdale 20, est de l'ordre de *D* = 6 mm. La vis 20 est réalisée en un matériau tel qu'un acier inoxydable, alliage platine-iridium ou autre matériau biocompatible, sous forme d'un fil de diamètre typique 0,3 à 0,8 mm enroulé en hélice avec un pas p = 0,8 à 4 mm (ces dimensions n'étant bien entendu aucunement limitatives et données purement à titre d'exemple). La vis 20 s'étend sur une région de contact dont la longueur L peut représenter 20 à 80 % de la longueur totale de la capsule 10.

De façon caractéristique, le diamètre intérieur de la vis d'ancrage hélicoïdale 20 est supérieur au diamètre extérieur du corps cylindrique 12, de manière à laisser subsister un intervalle libre e typiquement de l'ordre de e = 0,1 à 1 mm sur toute la longueur de la région de contact 22.

Le corps cylindrique 12 porte, dans l'exemple illustré, une électrode sectorielle 34 (Figure 2). En effet, afin de préserver la performance de stimulation, il est souhaitable de limiter la surface de l'électrode, ce qui peut se faire en limitant l'ouverture angulaire de celle-ci ("électrode sectorielle"). Pour pouvoir assurer au moment de l'implantation une bonne orientation de cette électrode sectorielle par rapport aux tissus, la position angulaire de la vis 20 par rapport à la capsule est ajustée pour que le centre de l'électrode 34 se situe à proximité immédiate de la dernière spire, qui se termine à l'intersection 36 de la trajectoire de la vis et de l'épaulement de butée 28, de préférence dans un secteur de 120° en amont par rapport à la génératrice de fin de filet. Ceci permet de garantir le contact physique de l'électrode sectorielle 34 avec les tissus contre lesquels sera implantée la capsule, et donc de préserver les performances électriques de la capsule.

En variante ou en complément d'une électrode sectorielle, il est possible de former une électrode directement sur la vis d'ancrage hélicoïdale 20, celle-ci étant alors réalisée en un matériau conducteur recouvert d'un revêtement électriquement isolant, à l'exception d'une ou plusieurs zones où le revêtement isolant aura été localement ablaté, de manière à laisser apparaitre le matériau conducteur de la vis qui, en contact avec les tissus, formeront une ou plusieurs électrodes de détection/stimulation.

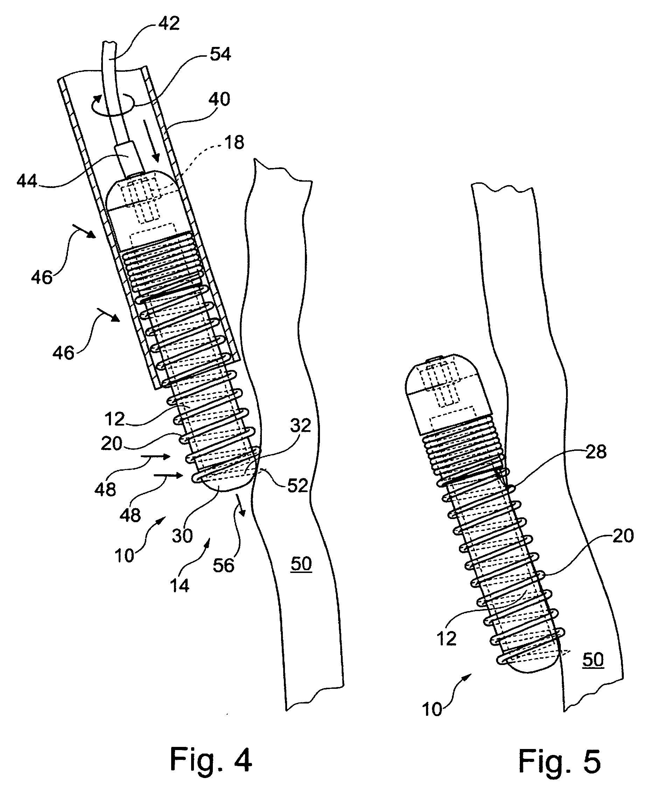

On va maintenant décrire, en référence aux Figures 4 et 5, la manoeuvre d'implantation d'une capsule 10 selon l'invention telle que celle que l'on vient de décrire.

Il est possible d'utiliser pour l'implantation un accessoire de guidage et de manoeuvre tel que celui de la demande française FR 13 56020 précitée, qui décrit un accessoire d'implantation mettant en oeuvre un cathéter téléorientable prolongé dans sa partie distale par un embout cylindrique de protection 40 contenant la capsule à implanter.

La capsule est initialement maintenue en position rétractée dans l'embout, avec la capsule reliée à un mandrin ou un sous-cathéter 42 inséré dans la lumière interne de l'accessoire implantation, la capsule 10 et le sous-cathéter 42 étant temporairement liés par un mécanisme débrayable 44 permettant de solidariser en rotation et en translation ces deux éléments, notamment pour permettre un vissage complet de la capsule dans les tissus.

La configuration télescopique de l'ensemble sous-cathéter 42/capsule 10 par rapport à l'embout 40 permet de projeter la capsule hors de cet embout et au-delà de celui-ci sur plusieurs centimètres, autorisant en toutes circonstances une approche complète et précise de la capsule jusqu'au site d'implantation.

L'embout 40 est manoeuvré de manière à le pousser contre le septum (pour une implantation sur un tel site) et la capsule est alors sortie de l'embout 40, l'ensemble embout-capsule étant plaqué (flèches 46) contre la paroi de manière que l'axe de la capsule forme un angle aigu avec la surface de la paroi.

Cette manoeuvre permet de maintenir un effort tangentiel (flèches 48) contre la paroi et d'assurer ainsi le piquage de l'extrémité 52 de la première spire 32 de la vis dans les tissus 50. La poursuite du mouvement de rotation imprimé à la capsule par le sous-cathéter 42 (flèche 54) génère alors une progression de la vis dans les tissus 50 (flèche 56), ceux-ci se trouvant coincés entre les spires de la vis et le corps de la capsule sur une génératrice de celle-ci.

La progression se poursuit jusqu'à ce que les tissus 50 viennent buter contre l'épaulement 28 (configuration illustrée Figure 5) formé sur le corps de la capsule. Cette butée va engendrer une augmentation brutale du couple de réaction sur le sous-cathéter 42, ce qui va provoquer le débrayage des moyens de solidarisation temporaire 44, protégeant les tissus de tout déchirement.

La configuration finale est celle illustrée Figure 5 : comme on peut le constater, la capsule est particulièrement bien fixée à la paroi 50, du fait du nombre important de spires assurant cette solidarisation. De plus, la pénétration étant, par conception, très peu profonde, les risques de perforation sont extrêmement faibles, même pour des parois fines comme par exemple la paroi libre de l'oreillette droite.

Ce système permet également la fixation sans risque d'une capsule de taille et/ou de poids important, avec un faible impact sur les tissus grâce à la répartition des contraintes de fixation sur plusieurs spires, le long d'une génératrice de la *capsule. A contrario,* avec les solutions conventionnelles mettant en oeuvre des moyens d'ancrage prolongeant la capsule à son extrémité distale, la contrainte est beaucoup plus concentrée, et accrue encore par l'effet de levier exercé sur la vis du fait de la distance entre le centre de gravité de la capsule et son extrémité distale.

Un autre avantage est le caractère parfaitement réversible du mode de fixation selon l'invention : si nécessaire, il est possible de retirer la capsule avec un endommagement minimal des tissus, simplement par une rotation exercée en sens inverse de celui de la vis d'ancrage hélicoïdale.

On notera enfin que le système de l'invention est applicable à une fixation sur des parois de nature très variée, aussi bien trabéculeuses que lisses, avec dans tous les cas la garantie d'une solidarisation robuste de la capsule à la paroi.

De nombreuses variantes de mise en oeuvre sont envisageables, l'invention n'étant bien entendu pas limitée à l'exemple décrit.

Ainsi, il est possible de donner à l'intervalle libre e entre l'intérieur de la vis et l'extérieur du corps tubulaire soit une valeur constante, soit une valeur variable dans la direction longitudinale de la capsule, si l'on souhaite par exemple limiter la contrainte mécanique exercée sur les tissus à proximité immédiate de l'électrode de stimulation. Cette variation de l'intervalle peut être également une variation vue dans un plan radial, l'intervalle e variant alors en fonction de la position angulaire du point de l'hélice de la vis sur un filet.

Le pas p de l'hélice de la vis d'ancrage 20 peut également être soit une valeur constante soit une valeur variable, se réduisant progressivement côté proximal pour accentuer le phénomène de pincement des tissus.

La capsule peut également être pourvue de marqueurs radio-opaques sectoriels pour permettre de contrôler sous amplificateur de brillance la bonne orientation de la capsule au moment de l'implantation, avec éventuellement un marqueur associé à l'électrode sectorielle de stimulation 34, ou constitué par celle-ci.

## Revendications

1. Une capsule intracardiaque (10), comprenant :
- un corps cylindrique (12) ;
- une vis d'ancrage hélicoïdale (20) solidaire du corps cylindrique, apte à pénétrer dans un tissu (50) d'une paroi d'une cavité du coeur ; et
- à une extrémité proximale (16) du corps cylindrique, des moyens (18) de solidarisation temporaire, en rotation et en translation, de la capsule à un accessoire d'implantation,
dans lequel :
- la vis d'ancrage hélicoïdale entoure le corps cylindrique sur au moins une partie (L) de la longueur de celui-ci, cette partie formant une région de contact (22) destinée à venir en contact avec la paroi de la cavité du coeur ;
- sur l'étendue de la région de contact, le diamètre externe du corps cylindrique est inférieur au diamètre interne de la vis d'ancrage hélicoïdale, de manière à laisser subsister un intervalle libre (e) entre le corps cylindrique et la vis d'ancrage hélicoïdale ; et
- la vis d'ancrage hélicoïdale est solidarisée (24, 26) au corps cylindrique au voisinage de l'extrémité proximale de la région de contact, et elle s'étend librement depuis cette extrémité proximale jusqu'à l'extrémité distale opposée,
**caractérisée en ce que**, ladite région de contact (22) s'étendant le long d'une génératrice du corps cylindrique :
- l'extrémité distale (14) du corps cylindrique comporte une surface arrondie atraumatique (30).

2. La capsule de la revendication 1, dans laquelle la vis d'ancrage (20) ne s'étend pas au-delà de l'extrémité de la surface arrondie atraumatique (30) en direction axiale.

3. La capsule de la revendication 1, dans laquelle la vis d'ancrage (20) s'étend au-delà de l'extrémité de la surface arrondie atraumatique (30) en direction axiale sur l'étendue d'une ou deux spires de vis.

4. La capsule de la revendication 1, dans laquelle la surface arrondie atraumatique (30) est conformée de manière à laisser exposée l'extrémité (32) de la vis d'ancrage (20) sur une longueur d'une ou deux spires frontales, de manière à pouvoir amorcer le vissage dans la paroi de la cavité du coeur par ces spires exposées.

5. La capsule de la revendication 1, dans laquelle ledit intervalle libre (e) subsistant entre le corps cylindrique et la vis d'ancrage hélicoïdale le long de la région de contact est compris entre 0,1 et 1 mm.

6. La capsule de la revendication 1, dans laquelle le corps cylindrique comprend en outre au voisinage de l'extrémité proximale de la région de contact un épaulement de butée (28) dont le diamètre est supérieur à celui de la région de contact, l'intervalle libre s'étendant en direction distale à partir de cet épaulement de butée.

7. La capsule de la revendication 1, comprenant en outre au moins une électrode sectorielle (34) de détection/stimulation disposée sur le corps cylindrique.

8. La capsule des revendications 6 et 7 prises en combinaison, dans laquelle l'électrode sectorielle (34) est disposée au voisinage de l'épaulement de butée (28).

9. La capsule de la revendication 1, dans laquelle la vis d'ancrage hélicoïdale est une vis en matériau électriquement conducteur recouvert d'un revêtement électriquement isolant à l'exception d'au moins une zone localement dénudée formant électrode de détection/stimulation.

10. La capsule de la revendication 1, dans laquelle l'intervalle libre (e) est un intervalle constant sur la longueur de la région de contact.

11. La capsule de la revendication 1, dans laquelle l'intervalle libre est un intervalle variable sur la longueur de la région de contact, cet intervalle étant accru côté proximal.

12. La capsule de la revendication 1, dans laquelle le pas (p) de la vis d'ancrage hélicoïdale est un pas constant.

13. La capsule de la revendication 1, dans laquelle le pas de la vis d'ancrage hélicoïdale est un pas variable, ce pas étant réduit côté proximal.

## Patentansprüche

1. Interkardiale Kapsel (10), die Folgendes umfasst:
- einen zylindrischen Körper (12);
- eine mit dem zylindrischen Körper fest verbundene Schneckenverankerungsschraube (20), die in ein Gewebe (50) einer Wand einer Herzkammer eindringen kann; und
- an einem proximalen Ende (16) des zylindrischen Körpers Mittel (18) zum vorübergehenden rotatorischen und translatorischen Befestigen der Kapsel an einer Implantationshilfe,
wobei:
- die Schneckenverankerungsschraube den zylindrischen Körper wenigstens auf einem Teil (L) seiner Länge umgibt, wobei dieser Teil einen Kontaktbereich (22) bildet, der dazu bestimmt ist, mit der Wand der Herzkammer in Kontakt zu gelangen;
- auf der Länge des Kontaktbereichs der Außendurchmesser des zylindrischen Körpers kleiner ist als der Innendurchmesser der Schneckenverankerungsschraube, derart, dass zwischen dem zylindrischen Körper und der Schneckenverankerungsschraube ein freier Zwischenraum (e) bestehen bleibt; und
- die Schneckenverankerungsschraube mit dem zylindrischen Körper in der Umgebung des proximalen Endes des Kontaktbereichs fest verbunden ist (24, 26) und sich von diesem proximalen Ende bis zu dem gegenüberliegenden distalen Ende frei erstreckt,
**dadurch gekennzeichnet, dass** sich der Kontaktbereich (22) längs einer Erzeugenden des zylindrischen Körpers erstreckt; und
- das distale Ende (14) des zylindrischen Körpers eine atraumatische abgerundete Oberfläche (30) aufweist.

2. Kapsel nach Anspruch 1, wobei sich die Verankerungsschraube (20) in axialer Richtung nicht über das Ende der atraumatischen abgerundeten Oberfläche (30) hinaus erstreckt.

3. Kapsel nach Anspruch 1, wobei sich die Verankerungsschraube (20) in axialer Richtung um eine oder zwei Windungen der Schraube über das Ende der atraumatischen abgerundeten Oberfläche (30) hinaus erstreckt.

4. Kapsel nach Anspruch 1, wobei die atraumatische abgerundete Oberfläche (30) so beschaffen ist, dass das Ende (32) der Verankerungsschraube (20) auf einer Länge von einer oder zwei vorderen Windungen frei liegt, derart, dass die Verschraubung in der Wand der Herzkammer durch diese frei liegenden Windungen beginnen kann.

5. Kapsel nach Anspruch 1, wobei der freie Zwischenraum (e), der zwischen dem zylindrischen Körper und der Schneckenverankerungsschraube längs des Kontaktbereichs bestehen bleibt, im Bereich von 0,1 bis 1 mm liegt.

6. Kapsel nach Anspruch 1, wobei der zylindrische Körper außerdem in der Umgebung des proximalen Endes des Kontaktbereichs eine Anschlagschulter (28) aufweist, deren Durchmesser größer ist als jener des Kontaktbereichs, wobei sich der freie Zwischenraum in distaler Richtung ausgehend von dieser Anschlagschulter erstreckt.

7. Kapsel nach Anspruch 1, die außerdem wenigstens eine sektorförmige Detektions-/Stimulationselektrode (34) umfasst, die an dem zylindrischen Körper angeordnet ist.

8. Kapsel nach den Ansprüchen 6 und 7 in Kombination, wobei die sektorförmige Elektrode (34) in der Umgebung der Anschlagschulter (28) angeordnet ist.

9. Kapsel nach Anspruch 1, wobei die Schneckenverankerungsschraube eine Schraube aus einem elektrisch leitenden Material ist, das mit Ausnahme wenigstens einer räumlich begrenzten abisolierten Zone, die die Detektions-/Stimulationselektrode bildet, mit einer elektrisch isolierenden Beschichtung beschichtet ist.

10. Kapsel nach Anspruch 1, wobei der freie Zwischenraum (e) ein Zwischenraum ist, der auf der Länge des Kontaktbereichs konstant ist.

11. Kapsel nach Anspruch 1, wobei der freie Zwischenraum ein Zwischenraum ist, der auf der Länge des Kontaktbereichs veränderlich ist, wobei dieser Zwischenraum zur proximalen Seite hin zunimmt.

12. Kapsel nach Anspruch 1, wobei die Steigung (p) der Schneckenverankerungsschraube eine konstante Steigung ist.

13. Kapsel nach Anspruch 1, wobei die Steigung der Schneckenverankerungsschraube eine veränderliche Steigung ist, wobei diese Steigung zur proximalen Seite hin abnimmt.

## Claims

1. An intracardiac capsule (10), comprising:
- a cylindrical body (12);
- a helical anchoring screw (20) integral with the cylindrical body, adapted to penetrate into a tissue (50) of a heart cavity wall; and
- at a proximal end (16) of the cylindrical body, means (18) for temporary fastening, in rotation and in translation, the capsule to an accessory of implantation,
wherein
- the helical anchoring screw surrounds the cylindrical body over at least one part (L) of the length thereof, this part forming a contact region (22) intended to come into contact with the heart cavity wall;
- over the extent of the contact region, the external diameter of the cylindrical body is lower than the internal diameter of the helical anchoring screw, so as to leave a free interval (e) between the cylindrical body and the helical anchoring screw; and
- the helical anchoring screw is fastened (24, 26) to the cylindrical body in the vicinity of the proximal end of the contact region, and it extends freely from this proximal end to the opposite distal end,
**characterized in that**, said contact region (22) extending along a generatrix of the cylindrical body:
- the distal end (14) of the cylindrical body includes an atraumatic round surface (30).

2. The capsule of claim 1, wherein the anchoring screw (20) does not extend beyond the end of the atraumatic round surface (30) in the axial direction.

3. The capsule of claim 1, wherein the anchoring screw (20) extends beyond the end of the atraumatic round surface (30) in the axial direction over one or two screw threads.

4. The capsule of claim 1, wherein the atraumatic round surface (30) is shaped so as to leave exposed the end (32) of the anchoring screw (20) over a length of one or two front threads, so as to be able to initiate the screwing into the heart cavity wall through these exposed threads.

5. The capsule of claim 1, wherein said free interval (e) remaining between the cylindrical body and the helical anchoring screw along the contact region is comprised between 0.1 and 1 mm.

6. The capsule of claim 1, wherein the cylindrical body further comprises, in the vicinity of the proximal end of the contact region, a stop shoulder (28) whose diameter is higher than that of the contact region, the free interval extending in the distal direction from this stop shoulder.

7. The capsule of claim 1, further comprising at least one sectoral detection/stimulation electrode (34) arranged on the cylindrical body.

8. The capsule of claims 6 and 7 taken in combination, wherein the sectoral electrode (34) is arranged in the vicinity of the stop shoulder (28).

9. The capsule of claim 1, wherein the helical anchoring screw is a screw made of an electrically conductive material covered with an electrically insulating coating except at least one locally-naked zone forming the detection/stimulation electrode.

10. The capsule of claim 1, wherein the free interval (e) is an interval that is constant over the length of the contact region.

11. The capsule of claim 1, wherein the free interval is an interval that is variable over the length of the contact region, this interval being increased on the proximal side.

12. The capsule of claim 1, wherein the pitch (p) of the helical anchoring screw is a constant pitch.

13. The capsule of claim 1, wherein the pitch of the helical anchoring screw is a variable pitch, this pitch being reduced on the proximal side.
